# EUROPEAN PATENT SPECIFICATION

(11) **EP 2 575 873 B1**
(45) Date of publication and mention of the grant of the patent: **30.12.2015**
(21) Application number: 11745827.3
(22) Date of filing: 01.06.2011
(51) Int. Cl.: A61K 39/145

(54) **CONCENTRATION AND LYOPHILIZATION OF INFLUENZA VACCINE ANTIGENS**
KONZENTRIERUNG UND LYOPHILISIERUNG VON INFLUENZA ANTIGENE ZUR IMPFUNG
CONCENTRATION ET LYOPHILISATION D'ANTIGENES D'INFLUENZA POUR VACCINS

(30) Priority: 01.06.2010 US 396720 P
(43) Date of publication of application: 10.04.2013
(73) Proprietor: Novartis AG, 4056 Basel (CH)
(72) Inventor: KOMMAREDDY, Sushma, Cambridge, MA 02139 (US); SCAMPINI, Amanda, Cambridge, MA 02139 (US); BAUDNER, Barbara, Cambridge, MA 02139 (US); O'HAGAN, Derek, Cambridge, MA 02139 (US); SINGH, Manmohan, Cambridge, MA 02139 (US)
(74) Representative: Carpmaels & Ransford LLP
(86) International application number: PCT/IB2011/001564
(87) International publication number: WO 2011/151726

(56) References cited:
- WO-A2-2004/084816
- KIM T H: "HIGH VIABILITY LYOPHILIZED BCG VACCINE PRODUCED BY DEEP CULTURE TECHNIQUE", APPLIED AND ENVIRONMENTAL MICROBIOLOGY, AMERICAN SOCIETY FOR MICROBIOLOGY, US, vol. 34, no. 5, 1 November 1977 (1977-11-01), pages 495-499, XP008145149, ISSN: 0099-2240
- KINYANJUI M W ET AL: "Enhanced transduction of antigen-stimulated T lymphocytes with recombinant retroviruses concentrated by centrifugal filtration", JOURNAL OF IMMUNOLOGICAL METHODS, ELSEVIER SCIENCE PUBLISHERS B.V.,AMSTERDAM, NL, vol. 314, no. 1-2, 31 July 2006 (2006-07-31), pages 80-89, XP028017495, ISSN: 0022-1759, DOI: 10.1016/J.JIM.2006.06.002 [retrieved on 2006-07-31]
- DATABASE EMBASE [Online] ELSEVIER SCIENCE PUBLISHERS, AMSTERDAM, NL EMB-1974081044, 1973 ISACHENKO V A ET AL: 'Experience of preparation and employment of inactivated antigens of influenza virus of various origins (Russian)' Database accession no. EMB-1974081044 & VOPROSY VIRUSOLOGII 1973, vol. 18, no. 4, 1973, pages 489-493, ISSN: 0507-4088

## Description

### TECHNICAL FIELD

This invention is in the field of processing antigen solutions for use in vaccines.

### BACKGROUND ART

During vaccine manufacture it is often the case that the concentration of antigen in a manufacturing bulk exceeds the concentration in a final patient formulation, and so the process involves a step in which the bulk is diluted. In some situations, however, it is necessary to increase the antigen concentration in an aqueous bulk, and the invention concerns processes for concentrating antigens. Useful processes should increase an antigen's concentration without destroying its immunogenicity.

One situation where antigen concentration is required is for new delivery techniques where only a small volume of material is delivered. For instance, vaccines can be delivered by microneedles [2,3] or by thin films or strips [1,14-17]. These techniques deliver much less volume than the typical intramuscular injection of 0.5ml but they may require the same amount of antigen, which will often require a more concentrated bulk antigen.

One existing concentration process which can increase the concentration of an individual influenza virus hemagglutinin (HA) from 125-500µg/ml to 14mg/ml involves tangential flow filtration (TFF) of a starting volume of aqueous material to a concentration of 10mg/ml, then lyophilisation, then reconstitution of the lyophilisate in a smaller aqueous volume than the starting volume. This process can be performed on three different monovalent HA bulks, and their reconstitution as a single trivalent aqueous composition can provide a final HA concentration of 42mg/ml.

It is an object of the invention to provide further and improved processes for increasing the concentration of antigen in a material for use in vaccine manufacture, and particularly for influenza vaccine manufacture, such as influenza vaccines which are not delivered by intramuscular injection.

### DISCLOSURE OF THE INVENTION

In contrast to an existing process in which antigen is concentrated using TFF, the antigen concentration procedure of the invention uses centrifugal filtration. Like the existing process, the concentrated material can then be lyophilised, and the lyophilised material can be reconstituted for further use.

Thus the invention provides a process for preparing a lyophilised vaccine antigen, comprising steps of (i) increasing the concentration of an antigen in a liquid composition including that antigen using centrifugal filtration, to provide a concentrated antigen, and (ii) lyophilising the concentrated antigen, to provide the lyophilised vaccine antigen; wherein the liquid composition is a bulk vaccine.

Also disclosed herein is a lyophilised vaccine antigen prepared by this process.

The lyophilised vaccine antigen can be used to formulate a vaccine, or can be reconstituted and then used to formulate a vaccine. This reconstitution is ideally in a smaller volume than the liquid composition's original volume, *i*.*e*. the volume at the start of step (i), and smaller than the concentrated antigen's pre-lyophilisation volume, *i.e.* the volume at the start of step (ii), as this again increases the antigen concentration. The reconstituted material can be used to formulate a vaccine.

Also disclosed herein is a vaccine formulated by this process.

The process is particularly useful for preparing a lyophilised influenza vaccine antigen, and this lyophilised influenza vaccine antigen is useful for formulating influenza vaccines.

### The antigen

Also disclosed herein is a process for concentrating antigens from various sources. The antigen may be from a bacterium, a virus, a fungus, or a parasite. Thus the vaccine may protect against disease caused by a bacterium, a virus, a fungus, and/or a parasite.

Typical bacteria for use with the process disclosed herein include, but are not limited to:
- *Bordetella,* such as *B. pertussis.*
- *Clostridia*, such as *C.tetani and C.botulinum*
- *Corynebacteria,* such as *C.diphtheriae.*
- *Pasteurella,* such as *Haemophrlus influenzae.*
- *Mycobacteria,* such as *M.tuberculossi, M.bovis* and the attenuated *Bacillus Calmette Guerin.*
- *Neisseria,* such as *N.nteningitidis* and *N. gonorrhoeae.*
- *Salmonella,* such as *S.typhi, S.paratyphi, S.typhinturium, S.enteritidis.*
- *Streptococci,* such as *S.pneumoniae* (pneumococcus), *S.agalactiae* and *S.pyogenes.*

Typical viruses for use with the process disclosed herein include, but are not limited to:
- *Orthomyxovirus,* such as an influenza A, B or C virus. Influenza A or B viruses may be interpandemic (annual/seasonal) strains, or from strains with the potential to cause a pandemic outbreak (*i.e.,* influenza strains with new hemagglutinin compared to a hemagglutinin in currently circulating strains, or influenza strains which are pathogenic in avian subjects and have the potential to be transmitted horizontally in the human population, or influenza strains which are pathogenic to humans). Depending on the particular season and on the nature of the strain, an influenza A virus may be derived from one or more of the following hemagglutinin subtypes: H1, H2, H3, H4, H5, H6, H7,H8, H9, H10, H11, H12, H13, H14, H15 or H16. More details are given below.
- *Paramyxoviridae* viruses, such as Pneumoviruses (RSV), Paramyxoviruses (PIV) and Morbilliviruses (Measles).
- *Pneumovirus* or *metapneumovirus,* for example respiratory syncytial virus (RSV), Bovine respiratory syncytial virus, Pneumonia virus of mice, and Turkey rhinotracheitis virus. Preferably, the Pneumovirus is RSV or human metapneumovirus (HMPV).
- *Paramyxovirus,* such as Parainfluenza virus (PIV) type 1, 2, 3 or 4, Mumps, Sendai viruses, Simian virus 5, Bovine parainfluenza virus and Newcastle disease virus. Preferably, the Paramyxovirus is PIV or Mumps.
- *Picornavirus*, such as Enteroviruses, Rhinoviruses, Heparnavirus, Cardioviruses and Aphthoviruses. Enteroviruses include Poliovirus types 1, 2 or 3, Coxsackie A virus types 1 to 22 and 24, Coxsackie B virus types 1 to 6, Echovirus (ECHO) virus) types 1 to 9, 11 to 27 and 29 to 34 and Enterovirus 68 to 71. Preferably, the Enterovirus is poliovirus *e.g.* a type 1 strain such as Mahoney or Brunenders, a type 2 strain such as MEF-I, or a type 3 strain such as Saukett. An example of a Heparnaviruses (also named Hepatoviruses) is Hepatitis A virus.
- *Togavirus,* such as a Rubivirus, an Alphavirus, or an Arterivirus. Rubiviruses, such as Rubella virus, are preferred. Useful alphaviruses for inactivation include aquatic alphaviruses, such as salmon pancreas disease virus and sleeping disease virus.
- *Flavivirus,* such as Tick-borne encephalitis (TBE), Dengue (types 1, 2, 3 or 4), Yellow Fever, Japanese encephalitis, West Nile encephalitis, St. Louis encephalitis, Russian spring-summer encephalitis, Powassan encephalitis.
- *Hepatitis C virus* (HCV).
- *Pestivirus,* such as Bovine viral diarrhea (BVDV), Classical swine fever (CSFV) or Border disease (BDV).
- *Hepadnavirus,* such as Hepatitis B virus.
- *Rhabdovirus,* such as a Lyssavirus (*e.g.* a rabies virus) and Vesiculovirus (VSV).
- *Caliciviridae,* such as Norwalk virus, and Norwalk-like Viruses, such as Hawaii Virus and Snow Mountain Virus, and Vesivirus, such as Vesicular Exanthema of Swine Virus.
- *Coronavirus*, such as a SARS, Human respiratory coronavirus, Avian infectious bronchitis (IBV), Mouse hepatitis virus (MHV), and Porcine transmissible gastroenteritis virus (TGEV).
- *Retrovirus,* such as an Oncovirus, a Lentivirus or a Spumavirus. An oncovirus may be HTLV-I, HTLV-2 or HTLV-3. A lentivirus may be SIV, HIV-1 or HIV-2.
- *Reovirus,* such as an Orthoreovirus, a Rotavirus, an Orbivirus, or a Coltivirus.
- *Parvovirus,* such as Parvovirus B 19, or Bocavirus.
- *Human Herpesvirus,* such as Herpes Simplex Viruses (HSV), Varicella-zoster virus (VZV), Epstein-Barr virus (EBV), Cytomegalovirus (CMV), Human Herpesvirus 6 (HHV6), Human Herpesvirus 7 (HHV7), and Human Herpesvirus 8 (HHV8).
- *Papovaviruses,* such as Papillomaviruses and Polyomaviruses. Papillomaviruses include HPV serotypes 1, 2, 4, 5, 6, 8, 11, 13, 16, 18, 31, 33, 35, 39, 41, 42, 47, 51, 57, 58, 63 and 65.
- *Adenoviridae,* including any of human adenoviruses A, B, C, D, E, F or G.

Disclosed herein are processes for preparing vaccines for viruses, and in particular viruses where the vaccine antigen is a viral surface glycoprotein. The invention is ideal for concentrating influenza virus hemagglutinin for preparing influenza vaccines, as described below in more detail. Steps (i) and (ii), followed by reconstitution, can provide an influenza vaccine antigen with a HA content of >5mg/ml, and even >10mg/ml.

### The liquid composition

A process of the invention increases the concentration of an antigen in a liquid composition, thereby providing a concentrated antigen for formulation purposes.

A preferred liquid composition is one which has never been lyophilised before step (ii). A preferred liquid composition is substantially free from lyoprotectants at the start of step (i). Thus a composition may be substantially free from exogenous sugar alcohols (in particular: sorbitol, mannitol, maltitol, erythritol, xylitol) and/or from exogenous disaccharides (in particular: sucrose, trehalose, maltose, lactulose, lactose, cellobiose). The combined concentration of (sorbitol, mannitol, maltitol, erythritol, xylitol, sucrose, trehalose, maltose, lactulose, lactose, cellobiose) in a liquid composition may thus be less than 10mg/ml (*i.e.* less than 1%) and is ideally less than 1mg/ml *e.g.* less than 0.1mg/ml.

In the invention, the liquid composition is a bulk vaccine *e.g.* containing enough antigen for at least 500 separate human unit doses of the vaccine.

The liquid composition may be monovalent (*i.e*. containing vaccine antigen for protecting against only one pathogen) or multivalent (*i.e*. containing vaccine antigen for protecting against more than one pathogen, which includes where there is more than one different non-cross-protective pathogen *e.g*. multiple meningococcal serogroups, or multiple influenza A virus hemagglutinin types).

The invention can be used with liquid samples having a variety of vaccine antigen concentrations. Typically the liquid sample will include a vaccine antigen at a concentration of at least 1 µg/ml.

### The concentration step

A process of the invention involves a step in which the concentration of an antigen is increased using centrifugal filtration.

Centrifugal filtration involves centrifugation of a liquid through a filter. The filter retains the antigen to be concentrated but does not retain solvent or smaller solutes. As the volume of the filtrate increases, the concentration of the antigen in the retentate also increases. This technique typically uses a fixed angle rotor. Various suitable centrifugal filtration devices are commercially available e.g. the products sold under trade marks Centricon™, Vivaspin™ and Spintek™. The cut-off of the filter will be selected such that the antigen of interest remains in the retentate.

Also disclosed herein is a process involving a step in which the concentration of an antigen is increased using ultrafiltration. Ultrafiltration involves the use of hydrostatic pressure to force a liquid against a semipermeable membrane. The filter retains the antigen to be concentrated but does not retain solvent or smaller solutes. Continued application of hydrostatic pressure causes the volume of the filtrate to increase, and thus the concentration of the antigen in the retentate also increases. Many ultrafiltration membranes are commercially available. The molecular weight cut-off (MWCO) of an ultrafiltration membrane determines which solutes can pass through the membrane (*i*.*e*. into the filtrate) and which are retained (*i.e.* in the retentate). The MWCO of the filter used with the invention will be selected such that substantially all of the antigen of interest remains in the retentate.

The concentration step preferably increases the concentration of the antigen of interest by at least *n*-fold, where *n* is 5, 6, 7, 8, 9, 10, 12, 15, 20, 25, 30, or more.

### The lyophilisation step

After antigen concentration, a process of the invention lyophilises the concentrated antigen to provide a lyophilised vaccine antigen. Kim (1977), Appl. Environ. Microbiol. 34(5):495-499 describes such a method, but the concentration step is carried out by ultrafiltration.

Lyophilisation typically involves three stages within a chamber: (a) freezing; (b) primary drying; and (c) secondary drying. Step (a) freezes the mobile water of the conjugate. In step (b) the chamber pressure is reduced (*e.g.* to ≤0.1 Torr) and heat is applied to the product to cause the frozen water to sublime. In step (c) the temperature is increased to desorb any bound water, such as water of crystallisation, until the residual water content falls to the desired level.

An initial step in a typical lyophilisation, before freezing occurs, is addition of a lyoprotectant. In some embodiments a lyoprotectant may have been added prior to concentration in step (i), but it is preferred to add it instead after concentration has occurred *i.e.* at the end of step (i) or at the start of step (ii). This makes it easier to control the amount of lyoprotectant which is present at the start of lyophilisation freezing.

Thus a process of the invention may involve a step of adding one or more lyoprotectants to the concentrated antigen. Suitable lyoprotectants include, but are not limited to, sugar alcohols (such as sorbitol, mannitol, maltitol, erythritol, xylitol) and disaccharides (such as sucrose, trehalose, maltose, lactulose, lactose, cellobiose). Sucrose and mannitol (or a mixture thereof) are preferred lyoprotectants for use with the invention.

After lyophilisation, a lyophilised vaccine antigen can be reconstituted. This reconstitution can use water (*e.g.* water for injection, wfi) or buffer (*e.g.* a phosphate buffer, a Tris buffer, a borate buffer, a succinate buffer, a histidine buffer, or a citrate buffer). Buffers will typically be included in the 5-20mM range. A phosphate buffer is preferred.

Step (i) concentrated the a first liquid volume of vaccine antigen, providing a composition with the same amount of antigen in a second (reduced) liquid volume. Step (ii) dried this concentrated material. This dried material can be reconstituted in a third liquid volume. If the third volume is greater than the first volume, the overall process has failed to concentrate the antigen. Similarly, if the third volume is greater than the second volume, the reconstitution step has gone backwards in terms of concentration. Thus the third volume is either equal to or, preferably, less than the second volume. Thus the lyophilisation/reconstitution steps can achieve a further antigen concentration. Embodiments where the third volume is equal to (or greater than) then second volume are still useful *e.g.* for buffer exchange, *etc*., but they are not preferred.

### Formulation

Lyophilised vaccine antigen can be used to formulate a vaccine, but will typically be reconstituted before doing so.

The invention can be used for preparing various vaccine formulations. The increased antigen concentration means that the invention is ideal for techniques which involve the delivery of small volumes of material to a patient. For instance, the invention is useful for preparing liquid vaccine formulations which have a unit dose volume of 0.1ml or less (*e.g.* for intradermal injection). The invention is also useful for preparing solid (including solid non-lyophilised) vaccine formulations, as these can require high antigen concentrations. As described in more detail below, suitable solid formulations include, but are not limited to, solid biodegradable microneedles, coated microneedles, and thin oral films. Thus a formulation step in a process of the invention may comprise: preparing a solid vaccine form from the lyophilised vaccine antigen.

Formulated vaccines of the invention will retain lyoprotectant(s) from the lyophilisation step. Thus a vaccine may comprise, for example, one or more of sorbitol, mannitol, maltitol, erythritol, xylitol, sucrose, trehalose, maltose, lactulose, lactose, and/or cellobiose.

Vaccines of the invention are ideally free from inulin.

### Solid biodegradable microneedles

One useful solid formulation which can be prepared using the invention is a solid biodegradable microneedle. These are typically not administered alone but, rather, multiple needles are administered simultaneously *e.g.* as a skin patch comprising a plurality of microneedles.

The microneedles are solid, such that they retain their structural integrity during storage and can penetrate a subject's skin when the patch is applied. The mechanical characteristics which are required for skin penetration depend on the organism in question, but they will usually have sufficient strength to penetrate human skin. Materials for forming suitable solid needles are readily available and these can be tested to determine appropriate concentrations *etc.* for any particular need.

The microneedles are biosolubic and biodegradable. Thus the solid material dissolves in the skin after the patch is applied, in contrast to the coated microneedles used in references 2 & 3 (see below). Having dissolved, the material will then be metabolised to give harmless end-products. The timescale for dissolving after applying the patch can vary, but dissolving will typically commence immediately after applying the patch (*e.g.* within 10 seconds) and may continue for *e.g.* up to 1 minute, 5 minutes, 10 minutes, 20 minutes, 30 minutes, 1 hour, 5 hours, 10 hours, or 24 hours, until the microneedle has fully dissolved. Materials with suitable *in vivo* dissolving kinetics are readily available and these can be varied and tested to determine appropriate concentrations *etc.* for any desired dissolution profile.

Suitable matrix materials for forming the microneedles will typically be biosoluble and biodegradable polymers, and these may comprise one or more carbohydrates. For example, the material may comprise a cellulose, a dextrin, a dextran, a disaccharide, a chitosan, a chitin, *etc.,* or mixtures thereof. Other GRAS materials may also be used. These materials can conveniently be combined with the vaccine antigen by including them in the liquid used to reconstitute the lyophilised vaccine antigen.

Suitable celluloses include, but are not limited to, cellulose, sodium carboxymethyl cellulose, hydroxypropyl cellulose, hydroxyethyl cellulose, and hydroxypropyl methylcellulose. Suitable dextrins include, but are not limited to, maltodextrin, cyclodextrin, amylodextrin, icodextrin, yellow dextrin, and white dextrins. Suitable disaccharides include, but are not limited to, sucrose, lactose, maltose, trehalose, turanose, and cellobiose. One suitable material for forming biosoluble and biodegradable microneedles is a dextrin/trehalose mixture.

The microneedles can penetrate the skin. They should be long enough to penetrate through the epidermis to deliver material into the dermis (*i.e*. intradermal delivery), but are ideally not so long that they can penetrate into or past the hypodermis. They will typically be 100-2500µm long *e.g.* between 1250-1750µm long, or about 1500µm. At the time of delivery the tip may penetrate the dermis, but the base of the needle may remain in the epidermis.

The microneedles can have various shapes and geometries. They will typically be tapered with a skin-facing point e.g. shaped as pyramids or cones. A tapered microneedle with a widest diameter of <500µm is typical.

A single patch will typically include a plurality of microneedles e.g. ≥ 10, ≥20, ≥30, ≥40, ≥50, ≥60, ≥70, ≥80, ≥90, ≥100, ≥200, ≥300, ≥400, ≥50, ≥750, ≥1000 or more per patch. Where a patch includes a plurality of microneedles, it may comprise a backing layer to which all of the microneedles are attached. A unitary backing layer with ≥20 projecting microneedles is typical. Where a patch includes a plurality of microneedles, these can be arranged in a regular repeating pattern or array, or may be arranged irregularly.

A patch will typically have an area of 3cm² or less, for example <2cm² or <1cm². A circular patch with a diameter of between 0.5cm and 1.5cm is useful.

The density of microneedles on a patch can vary, but may be ≥10cm⁻², ≥20cm⁻², ≥30cm⁻², ≥40cm⁻². ≥50cm⁻², ≥60cm⁻², ≥70cm⁻², ≥80cm⁻² or more.

A patch of the invention has a skin-facing inner face and an environment-facing outer face. The inner face may include an adhesive to facilitate adherence to a subject's skin. When present, it is preferably not present on the microneedles themselves *i.e.* the microneedles are adhesive-free. Rather than have adhesive on the inner face, a patch may have an additional backing which provides an outer adhesive margin for adhering the patch to skin *e.g.* as seen in sticking plasters or nicotine patches.

Patches as described above can be made by following the techniques and guidance in references 4-8. For instance, a mold with 1.5mm-long microneedle cavities can be prepared. A matrix material of dextrin and trehalose can be combined with an influenza vaccine and this aqueous material can be centrifugally cast in the mold to form an array of solid microneedles. A cellulose gel can then be cast over the matrix/vaccine mixture *(e.g.* which mixture has formed a film) to form a backing layer on the patch. When this backing layer has dried, it can be removed to give a patch from which the solid microneedles project. Thus a formulation step in a process of the invention may comprise: (a) mixing a biosoluble and biodegradable matrix material with the vaccine antigen, usually by reconstituting a lyophilised vaccine antigen; and (b) adding the mixture from step (a) to a mold containing cavities for forming microneedles. It may further comprise: (c) letting the mixture set in the mold, to form solid microneedles; (d) optionally, applying material to the set microneedles to provide a backing layer; and (e) removing the microneedles (and optional backing layer) from the mold.

Patches may be packaged into individual pouches *e.g.* sealed under nitrogen, then heat sealed. They should be stored carefully to avoid damage to the microneedles.

### Coated microneedles

Another useful solid formulation which can be prepared using the invention is a coated microneedle. These are typically not administered alone but, rather, multiple needles are administered simultaneously *e.g.* via a plurality of microneedles. One suitable product is marketed under the trade name of Macroflux™ (Zosano).

The microneedles are solid, such that they retain their structural integrity during storage and can penetrate a subject's skin. The mechanical characteristics which are required for skin penetration depend on the organism in question, but they will usually have sufficient strength to penetrate human skin. The microneedles are solid and remain intact after insertion into a patient's skin (in contrast to the biodegradable microneedles discussed above). Materials for forming suitable solid needles are readily available and these can be tested and selected for any particular need *e.g*. metals (such as stainless steel) or polymers (such as polycarbonate, ideally medical grade). Metal needles can be fabricated by using laser cutting and electro-polishing [9]. Polymer needles can be fabricated by microreplication and/or micromolding (including injection molding). Suitable microneedles are disclosed in references 2, 3, and 9-13.

An antigen of the invention can be coated onto the microneedles. This coating can be achieved by a simple process such as dip-coating *e.g.* involving a dipping step then a drying step (*e.g.* by evaporation), with repetition as required. Other useful coating techniques are disclosed in reference 11. Thus a formulation step in a process of the invention may comprise: applying the lyophilised vaccine antigen, or a reconstituted form thereof, to the surface of one or more solid microneedles to provide a coated microneedle device for injection of the vaccine.

A coating solution for applying to the needles can include one or more biosoluble and biodegradable matrix materials, and these may comprise one or more carbohydrates. For example, the material may comprise a cellulose, a dextrin, a dextran, a disaccharide, a chitosan, a chitin, *etc.,* or mixtures thereof. Other GRAS materials may also be used. Suitable celluloses, dextrins and disaccharides are listed above. These materials can conveniently be combined with the vaccine antigen by including them in the liquid used to reconstitute the lyophilised vaccine antigen.

Thus a formulation step in a process of the invention may comprise: (a) mixing a biosoluble and biodegradable matrix material with the vaccine antigen, usually by reconstituting a lyophilised vaccine antigen; and (b) applying the mixture from step (a) to the surface of one or more solid microneedles to provide a coated microneedle device for injection of the vaccine. Coating may be enhanced by using one or more "deposition enhancing components" as described in reference 11.

The applying steps discussed above may comprise an application sub-step followed by a drying sub-step, and this pair of sub-steps can be performed once or more than once *e.g.* 1, 2, 3, 4, 5, 6, 7, 8, 9, 10 or more times.

Microneedles in the device can penetrate the skin when applied. They should be long enough to penetrate through the epidermis to deliver material into the dermis (*i*.*e*. intradermal delivery), but are ideally not so long that they can penetrate into or past the hypodermis. They will typically be 100-2500µm long *e.g.* between 250-750µm long, or about 1500µm. At the time of delivery the tip may penetrate the dermis, but the base of the needle may remain in the epidermis. The needles can be applied to a patient's skin for between 30 seconds and 30 minutes, and then be removed.

The microneedles can have various shapes and geometries. They will typically be tapered with a skin-facing point *e.g*. shaped as pyramids or cones. A tapered microneedle with a widest diameter of <500µm is typical.

A microneedle device will typically include a plurality of microneedles *e.g.* ≥10, ≥20, ≥30, ≥40, ≥50, ≥60, ≥70, ≥80, ≥90, ≥100, ≥200, ≥300, ≥400, ≥50, ≥750, ≥1000, ≥1500, ≥2000 or more per device (for example, 300-1500 per device). Where a device includes a plurality of microneedles, these will typically all be attached to a unitary backing layer. Where a device includes a plurality of microneedles, these can be arranged in a regular repeating pattern or array, or may be arranged irregularly.

A microneedle device will typically have an area of 3cm² or less, for example <2cm² or <1cm². A circular device with a diameter of between 0.5cm and 1.5cm is useful.

The density of microneedles can vary, but may be ≥10cm⁻², ≥20cm⁻², ≥30cm⁻², ≥40cm⁻², ≥50cm⁻², ≥60cm⁻², ≥70cm⁻², ≥80cm⁻² or more. A device with 2mm between each microneedle, and a density of 14 microneedles/cm², is useful.

A microneedle device has a skin-facing inner face and an environment-facing outer face. The inner face may include an adhesive to facilitate adherence to a subject's skin. When present, it is preferably not present on the microneedles themselves *i.e.* the microneedles are adhesive-free. Rather than have adhesive on the inner face, a device may have an additional backing which provides an outer adhesive margin for adhering the device to skin.

A microneedle device may be packaged into individual pouches *e.g.* sealed under nitrogen, then heat sealed. They should be stored carefully to avoid damage to the microneedles.

### Thin films

Another useful solid formulation which can be prepared using the invention is a thin film, such as a thin oral film. These films wet and dissolve quickly upon contact with saliva and buccal tissue, therefore releasing the vaccine antigen in the mouth. The main component of these thin films is typically one or more hydrophilic polymer(s), which can have good mucoadhesive properties to provide strong adhesion to buccal tissue until complete dissolution. Similar films can be used for non-oral delivery *e.g.* for transcutaneous delivery as disclosed in reference 14.

Suitable thin films are typically 10-500µm thick when initially applied *e.g.* 75-150µm thick. Their other dimensions can be suitable to fit into a patient's mouth *e.g.* into an adult human mouth or into am infant human mouth.

One suitable type of film is disclosed in reference 15. This film comprises a mucoadhesive bilayer film with (i) Noveon and Eudragit S-100 as a mucoadhesive layer and (ii) a pharmaceutical wax as an impermeable backing layer. Further details of these films are in reference 16.

Another suitable type of film is disclosed in reference 17. This film comprises: (a) one or more water-soluble polymers; (b) one or more mucoadhesive polymers; (c) a vaccine antigen encapsulated within microparticles. Suitable water-soluble polymers include, but are not limited to: pullulan, hydroxypropyl cellulose, polyvinyl pyrrolidone, carboxymethyl cellulose, polyvinyl alcohol, sodium alginate, polyethylene glycol, xanthan gum, tragacanth gum, guar gum, acacia gum, Arabic gum, polyacrylic acid, methylmethacrylate copolymer, carboxyvinyl polymer, amylase, high amylase starch, hydroxypropylated high amylase starch, dextrin, pectin, chitin, levan, elsinan, collagen, gelatin, zein, gluten, soy protein isolate, whey protein isolate, and casein. Suitable mucoadhesive polymers include, but are not limited to: chitosan, hyaluronate, alginate, poly(acrylic acid), poly(methacrylic acid), poly(L-lysine), poly(ethyleneimine), poly(ethylene oxide), poly(2-hydroxyethyl methacrylate), and derivatives or copolymers thereof. Useful microparticles are made of a material which releases the particle's encapsulated contents (*i.e.* the vaccine antigen) while still present in the mouth.

The film in reference 14 comprises a cationic poly(β-amino ester) for transcutaneous delivery.

An oral film useful with the invention may include a flavouring agent to make the vaccine more palatable during administration.

Thin films can be made a variety of processes, including but not limited to: solvent casting; hot-melt extrusion; solid dispersion extrusion; and rolling.

A formulation step in a process of the invention may thus comprise: (a) mixing the vaccine antigen, usually by reconstituting a lyophilised vaccine antigen, with one or more orally-soluble polymers; and (b) forming a film using the mixture from step (a) to provide a thin film suitable for buccal administration of the vaccine.

A formulation step in a process of the invention may comprise: (a) mixing the vaccine antigen, usually by reconstituting a lyophilised vaccine antigen, with one or more topically-soluble polymers, such as a poly(β-amino ester); and (b) forming a film using the mixture from step (a) to provide a thin film suitable for transcutaneous administration of the vaccine.

These films may be packaged into individual unit dose pouches *e.g*. sealed under nitrogen, then heat sealed. The pouches should be water-tight to keep the films dry during storage.

### Use in methods of treatment, and administration of the vaccine

Formulated vaccines of the invention can be delivered to a subject *e.g.* via their skin, via their buccal tissue, *etc.* Thus the invention provides a formulated vaccine of the invention for use in a method of raising an immune response in a subject, comprising the step of administering a formulated vaccine of the invention to the subject. This might involve *e.g*. applying a microneedle patch or device to the subject's skin, such that the microneedles penetrate the subject's dermis, or applying a thin film to the subject's buccal tissue or tongue.

The invention also provides a lyophilised antigen for use in a method of vaccinating a subject. The invention also provides the use of lyophilised antigen in the manufacture of a medicament for raising an immune response in a subject.

The invention also provides a reconstituted lyophilised antigen for use in a method of vaccinating a subject. The invention also provides the use of reconstituted lyophilised antigen in the manufacture of a medicament for raising an immune response in a subject.

Vaccine products are suitable for administering vaccines to human or non-human animal subjects The immune response raised by these methods and uses will generally include an antibody response, preferably a protective antibody response.

Microneedle patches or devices may be applied to the skin by simple manual application (*e.g*. as with a sticking plaster or with known skin patches) or may be applied using a spring-driven injector.

Vaccines prepared according to the invention may be used to treat both children and adults.

Treatment can be by a single dose schedule or a multiple dose schedule. Multiple doses may be used in a primary immunisation schedule and/or in a booster immunisation schedule. Multiple doses will typically be administered at least 1 week apart (*e.g.* about 2 weeks, about 3 weeks, about 4 weeks, about 6 weeks, about 8 weeks, about 10 weeks, about 12 weeks, about 16 weeks, *etc.*).

### Influenza vaccination

Processes of the invention are ideal for preparing influenza vaccines. Various forms of influenza virus vaccine are currently available (*e.g.* see chapters 17 & 18 of reference 18) and current vaccines are based either on inactivated or live attenuated viruses. Inactivated vaccines (whole virus, split virion, or surface antigen) are administered by intramuscular or intradermal injection, whereas live vaccines are administered intranasally. The invention can be used with all of these vaccine forms.

Some embodiments of the invention use a surface antigen influenza vaccine (inactivated). Such vaccines contain fewer viral components than a split or whole virion vaccine. They include the surface antigens hemagglutinin and, typically, also neuraminidase. Processes for preparing these proteins in purified form from influenza viruses are well known in the art. The FLUVIRIN™, AGRIPPAL™ and INFLUVAC™ products are examples of surface antigen influenza vaccines.

Where the invention uses a surface antigen influenza vaccine, this virus may have been grown in eggs or in cell culture (see below). The current standard method for influenza virus growth for vaccines uses embryonated SPF hen eggs, with virus being purified from the egg contents (allantoic fluid). If egg-based viral growth is used then one or more amino acids may be introduced into the allantoid fluid of the egg together with the virus [24]. Virus is first grown in eggs. It is then harvested from the infected eggs. Virions can be harvested from the allantoic fluid by various methods. For example, a purification process may involve zonal centrifugation using a linear sucrose gradient solution that includes detergent to disrupt the virions. Antigens may then be purified, after optional dilution, by diafiltration. Chemical means for inactivating a virus include treatment with an effective amount of one or more of the following agents: detergents, formaldehyde, β-propiolactone, methylene blue, psoralen, carboxyfullerene (C60), binary ethylamine, acetyl ethyleneimine, or combinations thereof. Non-chemical methods of viral inactivation are known in the art, such as for example UV light or gamma irradiation.

Some embodiments of the invention can use whole virus, split virus, virosomes, live attenuated virus, or recombinant hemagglutinin. These vaccines can easily be distinguished from surface antigen vaccines by testing their antigens *e.g.* for the presence of extra influenza virus proteins.

Whole inactivated virus can be obtained by harvesting virions from virus-containing fluids (*e.g*. obtained from eggs or from culture medium) and then treating them as described above.

Split virions are obtained by treating purified virions with detergents (*e.g*. ethyl ether, polysorbate 80, deoxycholate, tri-*N*-butyl phosphate, Triton X-100, Triton N101, cetyltrimethylammonium bromide, Tergitol NP9, *etc*.) to produce subvirion preparations, including the 'Tween-ether' splitting process. Methods of splitting influenza viruses, for example are well known in the art *e.g.* see refs. 19-24, *etc.* Splitting of the virus is typically carried out by disrupting or fragmenting whole virus, whether infectious or non-infectious with a disrupting concentration of a splitting agent. The disruption results in a full or partial solubilisation of the virus proteins, altering the integrity of the virus. Preferred splitting agents are non-ionic and ionic (*e.g.* cationic) surfactants *e.g.* alkylglycosides, alkylthioglycosides, acyl sugars, sulphobetaines, betains, polyoxyethylene-alkylethers, N,N-dialkyl-Glucamides, Hecameg, alkylphenoxy-polyethoxyethanols, NP9, quaternary ammonium compounds, sarcosyl, CTABs (cetyl trimethyl ammonium bromides), tri-*N*-butyl phosphate, myristyltrimethylammonium salts, lipofectin, lipofectamine, and DOT-MA, the octyl- or nonylphenoxy polyoxyethanols (*e.g*. the Triton surfactants, such as Triton X-100 or Triton N101), polyoxyethylene sorbitan esters (the Tween surfactants), polyoxyethylene ethers, polyoxyethlene esters, *etc.* One useful splitting procedure uses the consecutive effects of sodium deoxycholate and formaldehyde, and splitting can take place during initial virion purification (e.g. in a sucrose density gradient solution). Thus a splitting process can involve clarification of the virion-containing material (to remove non-virion material), concentration of the harvested virions (*e.g*. using an adsorption method, such as CaHPO₄ adsorption), separation of whole virions from non-virion material, splitting of virions using a splitting agent in a density gradient centrifugation step (*e.g*. using a sucrose gradient that contains a splitting agent such as sodium deoxycholate), and then filtration (*e.g*. ultrafiltration) to remove undesired materials. Split virions can usefully be resuspended in sodium phosphate-buffered isotonic sodium chloride solution. Examples of split vaccines are the BEGRIVAC™, INTANZA™, FLUARIX™, FLUZONE™ and FLUSHIELD™ products.

Virosomes are nucleic acid free viral-like liposomal particles [25]. They can be prepared by solubilization of virus with a detergent followed by removal of the nucleocapsid and reconstitution of the membrane containing the viral glycoproteins. An alternative method for preparing virosomes involves adding viral membrane glycoproteins to excess amounts of phospholipids, to give liposomes with viral proteins in their membrane.

Live attenuated viruses are obtained from viruses (grown in eggs or in cell culture), but the viruses are not inactivated. Rather, the virus is attenuated ("att") *e.g.* so as not to produce influenza-like illness in a ferret model of human influenza infection. It may also be a cold-adapted ("ca") strain *i.e.* it can replicate efficiently at 25°C, a temperature that is restrictive for replication of many wildtype influenza viruses. It may also be temperature-sensitive ("ts") *i.e.* its replication is restricted at temperatures at which many wild-type Influenza viruses grow efficiently (37-39°C). The cumulative effect of the ca, ts, and att phenotype is that the virus in the attenuated vaccine can replicate in the nasopharynx to induce protective immunity in a typical human patient, but it does not cause disease *i.e.* it is safe for general administration to the target human population. These viruses can be prepared by purifying virions from virion-containing fluids *e.g.* after clarification of the fluids by centrifugation, then stabilization with buffer (*e.g.* containing sucrose, potassium phosphate, and monosodium glutamate). Live vaccines include the FLUMIST™ product. Although live vaccines can be used with the invention, it is preferred to use non-live vaccines.

As an alternative to using antigens obtained from virions, haemagglutinin can be expressed in a recombinant host (*e.g.* in an insect cell line, such as Sf9, using a baculovirus vector) and used in purified form [26-28] or in the form of virus-like particles (VLPs; e.g. see references 29 & 30).

Some embodiments of the invention use influenza vaccine prepared from viruses which were grown in cell culture, rather than in eggs. When cell culture is used, the viral growth substrate will typically be a cell line of mammalian origin. Suitable mammalian cells of origin include, but are not limited to, hamster, cattle, primate (including humans and monkeys) and dog cells. Various cell types may be used, such as kidney cells, fibroblasts, retinal cells, lung cells, *etc.* Examples of suitable hamster cells are the cell lines having the names BHK21 or HKCC. Suitable monkey cells are *e.g.* African green monkey cells, such as kidney cells as in the Vero cell line. Suitable dog cells are *e.g.* kidney cells, as in the MDCK cell line. Thus suitable cell lines include, but are not limited to: MDCK; CHO; 293T; BHK; Vero; MRC-5; PER.C6; WI-38; *etc..* Preferred mammalian cell lines for growing influenza viruses include: MDCK cells [31-34], derived from Madin Darby canine kidney; Vero cells [35-37], derived from African green monkey (*Cercopithecus aethiops*) kidney; or PER.C6 cells [38], derived from human embryonic retinoblasts. These cell lines are widely available *e.g.* from the American Type Cell Culture (ATCC) collection, from the Coriell Cell Repositories, or from the European Collection of Cell Cultures (ECACC). For example, the ATCC supplies various different Vero cells under catalog numbers CCL-81, CCL-81.2, CRL-1586 and CRL-1587, and it supplies MDCK cells under catalog number CCL-34. PER.C6 is available from the ECACC under deposit number 96022940. As a less-preferred alternative to mammalian cell lines, virus can be grown on avian cell lines [*e.g.* refs. 39-41], including cell lines derived from ducks (*e.g.* duck retina) or hens. Examples of avian cell lines include avian embryonic stem cells [39,42] and duck retina cells [40]. Suitable avian embryonic stem cells, include the EBx cell line derived from chicken embryonic stem cells, EB45, EB14, and EB14-074 [43]. Chicken embryo fibroblasts (CEF) may also be used.

The most preferred cell lines for growing influenza viruses are MDCK cell lines. The original MDCK cell line is available from the ATCC as CCL-34, but derivatives of this cell line may also be used. For instance, reference 31 discloses a MDCK cell line that was adapted for growth in suspension culture ('MDCK 33016', deposited as DSM ACC 2219). Similarly, reference 44 discloses a MDCK-derived cell line that grows in suspension in serum-free culture ('B-702', deposited as FERM BP-7449). Reference 45 discloses non-tumorigenic MDCK cells, including 'MDCK-S' (ATCC PTA-6500), 'MDCK-SF101' (ATCC PTA-6501), 'MDCK-SF102' (ATCC PTA-6502) and 'MDCK-SF103' (PTA-6503). Reference 46 discloses MDCK cell lines with high susceptibility to infection, including 'MDCK.5F1' cells (ATCC CRL-12042). Any of these MDCK cell lines can be used.

Where virus has been grown on a mammalian cell line then products of the invention will advantageously be free from egg proteins (*e.g.* ovalbumin and ovomucoid) and from chicken DNA, thereby reducing potential allergenicity.

Hemagglutinin in cell-derived products of the invention can have a different glycosylation pattern from the patterns seen in egg-derived viruses. Thus the HA (and other glycoproteins) may include glycoforms that are not seen in chicken eggs. Useful HA includes canine glycoforms.

The absence of egg-derived materials and of chicken glycoforms provides a way in which vaccine prepared from viruses grown in cell culture can be distinguished from egg-derived products.

Where virus has been grown on a cell line then the culture for growth, and also the viral inoculum used to start the culture, will preferably be free from (*i.e*. will have been tested for and given a negative result for contamination by) herpes simplex virus, respiratory syncytial virus, parainfluenza virus 3, SARS coronavirus, adenovirus, rhinovirus, reoviruses, polyomaviruses, birnaviruses, circoviruses, and/or parvoviruses [47]. Absence of herpes simplex viruses is particularly preferred.

For growth on a cell line, such as on MDCK cells, virus may be grown on cells in suspension [31, 48, 49] or in adherent culture. One suitable MDCK cell line for suspension culture is MDCK 33016 (deposited as DSM ACC 2219). As an alternative, microcarrier culture can be used.

Cell lines supporting influenza virus replication are preferably grown in serum-free culture media and/or protein free media. A medium is referred to as a serum-free medium in the context of the present invention in which there are no additives from serum of human or animal origin. Protein-free is understood to mean cultures in which multiplication of the cells occurs with exclusion of proteins, growth factors, other protein additives and non-serum proteins, but can optionally include proteins such as trypsin or other proteases that may be necessary for viral growth. The cells growing in such cultures naturally contain proteins themselves.

Cell lines supporting influenza virus replication are preferably grown below 37°C [50] during viral replication e.g. 30-36°C, at 31-35°C, or at 33±1°C.

The method for propagating virus in cultured cells generally includes the steps of inoculating the cultured cells with the strain to be cultured, cultivating the infected cells for a desired time period for virus propagation, such as for example as determined by virus titer or antigen expression (*e.g.* between 24 and 168 hours after inoculation) and collecting the propagated virus. The cultured cells are inoculated with a virus (measured by PFU or TCID₅₀) to cell ratio of 1:500 to 1:1, preferably 1:100 to 1:5, more preferably 1:50 to 1:10. The virus is added to a suspension of the cells or is applied to a monolayer of the cells, and the virus is absorbed on the cells for at least 60 minutes but usually less than 300 minutes, preferably between 90 and 240 minutes at 25°C to 40°C, preferably 28°C to 37°C. The infected cell culture (*e.g.* monolayers) may be removed either by freeze-thawing or by enzymatic action to increase the viral content of the harvested culture supernatants. The harvested fluids are then either inactivated or stored frozen. Cultured cells may be infected at a multiplicity of infection ("m.o.i.") of about 0.0001 to 10, preferably 0.002 to 5, more preferably to 0.001 to 2. Still more preferably, the cells are infected at a m.o.i of about 0.01. Infected cells may be harvested 30 to 60 hours post infection. Preferably, the cells are harvested 34 to 48 hours post infection. Still more preferably, the cells are harvested 38 to 40 hours post infection. Proteases (typically trypsin) are generally added during cell culture to allow viral release, and the proteases can be added at any suitable stage during the culture.

A vaccine product including vaccine prepared from cell culture preferably contains less than 10ng (preferably less than 1ng, and more preferably less than 100pg) of residual host cell DNA per dose, although trace amounts of host cell DNA may be present.

It is preferred that the average length of any residual host cell DNA is less than 500bp e.g. less than 400bp, less than 300bp, less than 200bp, less than 100bp, *etc.*

Contaminating DNA can be removed during vaccine preparation using standard purification procedures *e.g.* chromatography, *etc.* Removal of residual host cell DNA can be enhanced by nuclease treatment *e.g.* by using a DNase. A convenient method for reducing host cell DNA contamination is disclosed in references 51 & 52, involving a two-step treatment, first using a DNase (*e.g.* Benzonase), which may be used during viral growth, and then a cationic detergent *(e.g.* CTAB), which may be used during virion disruption. Treatment with an alkylating agent, such as β-propiolactone, can also be used to remove host cell DNA, and advantageously may also be used to inactivate virions [53].

Some embodiments of the invention use a monovalent influenza vaccine *(i.e.* it includes hemagglutinin antigen from a single influenza virus strain) but in some embodiments it may be a multivalent vaccine, such as a bivalent vaccine, trivalent vaccine, a tetravalent vaccine, or a >4-valent vaccine (*i.e*. including hemagglutinin from more than four different influenza virus strains). Monovatent and multivalent vaccines are readily distinguished by testing for multiple HA types, by amino acid sequencing, *etc.*

A monovalent vaccine is particularly useful for immunising against a pandemic or potentially-pandemic strain, either during a pandemic or in a pre-pandemic situation. Characteristics of these strains are: (a) they contain a new hemagglutinin compared to the hemagglutinins in currently-circulating human strains, *i.e.* one that has not been evident in the human population for over a decade *(e.g.* H2), or has not previously been seen at all in the human population *(e.g.* H5, H6 or H9, that have generally been found only in bird populations), such that the human population will be immunologically naïve to the strain's hemagglutinin; (b) they are capable of being transmitted horizontally in the human population; and (c) they are pathogenic to humans. These strains may have any of influenza A HA subtypes H1, H2, H3, H4, H5, H6, H7, H8, H9, H10, H11, H12, H13, H14, H15 or H16. A virus with H5 hemagglutinin type is preferred for immunizing against pandemic influenza, or a H2, H7 or H9 subtype. The invention may protect against one or more of influenza A virus NA subtypes N1, N2, N3, N4, N5, N6, N7, N8 or N9. Thus possible strains include H5N1. H5N3, H9N2, H2N2, H7N1 and H7N7, and any other emerging potentially pandemic strains.

A multivalent vaccine is more typical in a seasonal setting *e.g.* a trivalent vaccine is typical, including hemagglutinins from two influenza A virus strains and one influenza B virus strain, such as from a H1N1 influenza A strain, a H3N2 influenza A virus strain, and an influenza B virus strain. A tetravalent vaccine is also useful [54] *e.g.* including antigens from two influenza A virus strains and two influenza B virus strains, or three influenza A virus strains and one influenza B virus strain. Thus a vaccine may be bivalent, trivalent, tetravalent, *etc.* Except for monovalent vaccines, it is usual to include hemagglutinin from both influenza A and influenza B virus strains. In vaccines including only two influenza A virus strains, these will usually be one H1 strain (e.g. a H1N1 strain) and one H3 strain (*e.g.* a H3N2 strain). In some embodiments, however, there may be one pandemic influenza A virus strain and one H1 strain, or one pandemic influenza A virus strain and one H3 strain.

Where a vaccine includes more than one strain of Influenza, the different strains are typically grown separately and are mixed after the viruses have been harvested and antigens have been prepared. Thus a process of the invention may include the step of mixing antigens from more than one influenza strain.

As described in reference 54, exemplary tetravalent vaccines can include hemagglutinin from two influenza A virus strains and two influenza B virus strains ('A-A-B-B'), or from three influenza A virus strains and one influenza B virus strain ('A-A-A-B').

Influenza B virus currently does not display different HA subtypes, but influenza B virus strains do fall into two distinct lineages. These lineages emerged in the late 1980s and have HAs which can be antigenically and/or genetically distinguished from each other [55]. Current influenza B virus strains are either B/Victoria/2/87-like or B/Yamagata/16/88-like. Where a vaccine of the invention includes two influenza B strains, this will usually be one B/Victoria/2/87-like strain and one B/Yamagata/16/88-like strain. These strains are usually distinguished antigenically, but differences in amino acid sequences have also been described for distinguishing the two lineages *e.g.* B/Yamagata/16/88-like strains often (but not always) have HA proteins with deletions at amino acid residue 164, numbered relative to the 'Lee40' HA sequence [56].

Preferred A-A-B-B vaccines include hemagglutinins from: (i) a H1N1 strain; (ii) a H3N2 strain; (iii) a B/Victoria/2/87-like strain; and (iv) B/Yamagata/16/88-like strain.

In vaccines including three influenza A virus strains, these will usually be one H1 strain (*e.g.* a H1N1 strain) and two H3 strains (*e.g.* two H3N2 strains). The two H3 strains will have antigenically distinct HA proteins *e.g.* one H3N2 strain that cross-reacts with A/Moscow/10/99 and one H3N2 strain that cross-reacts with A/Fujian/411/2002. The two H3 strains may be from different clades (clades A, B and C of H3N2 strains are disclosed in reference 57). In some embodiments, however, one of these strains (*i*.*e*. H1, or one of the two H3 strains) may be replaced by a pandemic strain.

Thus one preferred A-A-A-B vaccine includes hemagglutinins from: (i) a H1N1 strain: (ii) a A/Moscow/10/99-like H3N2 strain; (iii) a A/Fujian/411/2002-like H3N2 strain; and (iv) an influenza B virus strain, which may be B/Victoria/2/87-like or B/Yamagata/16/88-like.

Another preferred A-A-A-B vaccine includes hemagglutinins from: (i) a H1N1 strain, (ii) a H3N2 strain, (iii) a H5 strain (*e.g.* a H5N1 strain) and (iv) an influenza B strain.

Another preferred A-A-A-B vaccine includes hemagglutinins from: (i) two different H1 strains, (ii) a H3N2 strain, and (iii) an influenza B strain.

Where antigens are present from two or more influenza B virus strains, at least two of the influenza B virus strains may have distinct hemagglutinins but related neuraminidases. For instance, they may both have a B/Victoria/2/87-like neuraminidase [58] or may both have a B/Yamagata/16/88-like neuraminidase. For instance, two B/Victoria/2/87-like neuraminidases may both have one or more of the following sequence characteristics: (1) not a serine at residue 27, but preferably a leucine; (2) not a glutamate at residue 44, but preferably a lysine; (3) not a threonine at residue 46, but preferably an isoleucine; (4) not a proline at residue 51, but preferably a serine; (5) not an arginine at residue 65, but preferably a histidine; (6) not a glycine at residue 70, but preferably a glutamate; (7) not a leucine at residue 73, but preferably a phenylalanine; and/or (8) not a proline at residue 88, but preferably a glutamine. Similarly, in some embodiments the neuraminidase may have a deletion at residue 43, or it may have a threonine; a deletion at residue 43, arising from a trinucleotide deletion in the NA gene, has been reported as a characteristic of B/Victoria/2/87-like strains, although recent strains have regained Thr-43 [58]. Conversely, of course, the opposite characteristics may be shared by two B/Yamagata/16/88-like neuraminidases *e.g.* S27, E44, T46, P51, R65, G70, L73, and/or P88. These amino acids are numbered relative to the 'Lee40' neuraminidase sequence [59]. Thus a A-A-B-B vaccine of the invention may use two B strains that are antigenically distinct for HA (one B/Yamagata/16/88-like, one B/Victoria/2/87-like), but are related for NA (both B/Yamagata/16/88-like, or both B/Victoria/2/87-like).

In some embodiments, the invention does not encompass a trivalent split vaccine containing hemagglutinin from each of A/New Caledonia/20/99 (H1N1), A/Wyoming/03/2003 (H3N2) and B/Jiangsu/10/2003 strains.

Strains whose antigens can usefully be included in the compositions include strains which are resistant to antiviral therapy (*e.g.* resistant to oseltamivir [60] and/or zanamivir), including resistant pandemic strains [61].

In some embodiments of the invention, a vaccine may include a small amount of mercury-based preservative, such as thiomersal or merthiolate. When present, such preservatives will typically provide less than 5µg/ml mercury, and lower levels are possible *e.g.* <1µg/ml, <0.5µg/ml. Preferred vaccines are free from thiomersal, and are more preferably mercury-free [23,62]. Such vaccines may include a non-mercurial preservative. Non-mercurial alternatives to thiomersal include 2-phenoxyethanol or α-tocopherol succinate [23]. Most preferably, a vaccine is preservative-free.

In some embodiments, a vaccine may include a stabilising amount of gelatin *e.g.* at less than 0.1%. In other embodiments, however, a vaccine is gelatin-free. The absence of gelatin can assure that the vaccine is safe in the small proportion of patients who are gelatin-sensitive [63,64].

In some embodiments, a vaccine may include one or more antibiotics *e.g.* neomycin, kanamycin, polymyxin B. In preferred embodiments, though, the vaccine is free from antibiotics.

In some embodiments, a vaccine may include formaldehyde. In preferred embodiments, though, the vaccine is free from formaldehyde.

As mentioned above, in some embodiments a vaccine may include egg components (*e.g.* ovalbumin and ovomucoid), but preferred embodiments are free from egg components.

The preparation of vaccines without the use of certain components and additives is disclosed in reference 65, thereby ensuring that these materials are not present even in residual amounts.

Hemagglutinin (HA) is the main immunogen in current inactivated influenza vaccines, and vaccine doses are standardised by reference to HA levels, typically measured by SRID. Existing vaccines typically contain about 15µg of HA per strain, although lower doses can be used *e.g.* for children, or in pandemic situations, or when using an adjuvant. Fractional doses such as ½ (*i.e.* 7.5µg HA per strain), ¼ and ⅛ have been used, as have higher doses (*e.g.* 3x or 9x doses [66,67]). These vaccines have a dosage volume of 0.5ml *i.e.* a typical HA concentration of 30µg/ml/strain. The trivalent INTANZA™ product contains 9µg of HA per strain in a 0.1 ml volume i.e. a HA concentration of 90µg/ml/strain, giving a total HA concentration of 270µg/ml.

Products of the present invention can include between 0.1 and 50µg of HA per influenza strain per dose, preferably between 0.1 and 50µg *e.g.* 1-20µg. Ideally a product has ≤16µg hemagglutinin per strain *e.g.* 1-15µg, 1-10µg, 1-7.5µg, 1-5µg, *etc.* Particular HA doses per strain include *e.g.* about 15, about 10, about 7.5, about 5, about 3.8, about 1.9, about 1.5, *etc.*

For live vaccines, dosing is measured by median tissue culture infectious dose (TCID₅₀) rather than HA content *e.g.* a TCID₅₀ of between 10⁶ and 10⁸ (preferably between 10^{6.5}-10^{7.5}) per strain per dose.

Influenza strains used with the invention may have a natural HA as found in a wild-type virus, or a modified HA. For instance, it is known to modify HA to remove determinants *(e.g.* hyper-basic regions around the HA1/HA2 cleavage site) that cause a virus to be highly pathogenic in avian species. The use of reverse genetics facilitates such modifications.

Vaccine products of the invention can include components in addition to the influenza vaccine antigens. As discussed above, for example, they can include a biosoluble and biodegradable matrix material, or an oral film polymer.

Vaccine products may include a detergent. The level of detergent can vary widely *e.g.* between 0.05-50 µg detergent per µg of HA ('µg/µg'). A low level of detergent can be used *e.g.* between 0.1-1µg/µg, or a high level can be used *e.g.* between 5-30µg/µg. The detergent may be a single detergent (*e.g.* polysorbate 80, or CTAB) or a mixture (*e.g.* both polysorbate 80 and CTAB). Preferred detergents are non-ionic, such as polysorbate 80 ('Tween 80') or octyl phenol ethoxylate ('Triton X100'). Polysorbate 80 may be present at between 0.05-50 µg polysorbate 80 per µg of HA *e.g.* between 0.1-1µg/µg, 0.1-0.8µg/µg, 0.1-0.5µg/µg, 5-40µg/µg, 5-30µg/µg, or 8-25µg/µg.

As mentioned above, some vaccine products may include preservatives such as thiomersal or 2-phenoxyethanol, but preferred vaccines are mercury- or preservative-free.

Vaccine products may include a physiological salt, such as a sodium salt. Sodium chloride (NaCl) is preferred, which may be present at between 1 and 20 mg/ml. Other salts that may be present include potassium chloride, potassium dihydrogen phosphate, disodium phosphate dehydrate, magnesium chloride, calcium chloride, *etc.*

Vaccine products may include one or more buffers. Typical buffers include: a phosphate buffer; a Tris buffer; a borate buffer; a succinate buffer; a histidine buffer (particularly with an aluminum hydroxide adjuvant); or a citrate buffer. Buffers will typically be included in the 5-20mM range.

Vaccine products are preferably sterile. Vaccine products are preferably non-pyrogenic *e.g.* containing <1 EU (endotoxin unit, a standard measure) per dose, and preferably <0.1 EU per dose. Vaccine products are preferably gluten-free.

Vaccine products can include immunostimulatory molecules. These can be mixed with antigen before preparing a patch. Suitable classes of immunostimulatory molecule include, but are not limited to: TLR3 agonists; TLR4 agonists; TLR5 agonists; TLR7 agonists; TLR8 agonists; TLR9 agonists; and CD I d agonists. Suitable immunostimulatory molecules include, but are not limited to: imidazoquinolines such as imiquimod ("R-837") [68,69] and resiquimod ("R-848") [70], or salts thereof *(e.g.* the hydrochloride salts); aminoalkyl glucosaminide phosphate derivatives, such as RC-529 [71,72]; α-glycosylceramides, such as α-galactosylceramide; 'ER 804057' from reference 73; E5564 [74,75]; *etc.*

Methods for assessing antibody responses, neutralising capability and protection after influenza virus vaccination are well known in the art. Human studies have shown that antibody titers against hemagglutinin of human influenza virus are correlated with protection (a serum sample hemagglutination-inhibition titer of about 30-40 gives around 50% protection from infection by a homologous virus) [76]. Antibody responses are typically measured by hemagglutination inhibition, by microneutralisation, by single radial immunodiffusion (SRID), and/or by single radial hemolysis (SRH). These assay techniques are well known in the art. Preferred vaccines satisfy I, 2 or 3 of the CPMP criteria for efficacy. In adults (18-60 years), these criteria are: (I) ≥70% seroprotection; (2) ≥40% seroconversion; and/or (3) a GMT increase of ≥2.5-fold. In elderly (>60 years), these criteria are: (1) >60% seroprotection; (2) >30% seroconversion; and/or (3) a GMT increase of >2-fold. These criteria are based on open label studies with at least 50 patients.

Influenza vaccines are currently recommended for use in pediatric and adult immunisation, from the age of 6 months. Thus a human subject may be less than 1 year old, 1-5 years old, 5-15 years old, 15-55 years old, or at least 55 years old. Preferred subjects for receiving the vaccines are the elderly (*e.g.* ≥50 years old, ≥60 years old, and preferably ≥65 years), the young (*e.g.* ≤5 years old), hospitalised subjects, healthcare workers, armed service and military personnel, pregnant women, the chronically ill, immunodeficient subjects, subjects who have taken an antiviral compound (*e.g.* an oseltamivir or zanamivir compound; see below) in the 7 days prior to receiving the vaccine, people with egg allergies and people travelling abroad. The vaccines are not suitable solely for these groups, however, and may be used more generally in a population. For pandemic strains, administration to all age groups is preferred.

Administration of more than one dose (typically two doses) is particularly useful in immunologically naïve patients *e.g.* for people who have never received an influenza vaccine before, or for vaccinating against a new HA subtype (as in a pandemic outbreak).

### Reconstitution using a buffer

In a further aspect, the invention provides a process for preparing a vaccine antigen, comprising steps of (i) increasing the concentration of an antigen in a liquid composition including that antigen using centrifugal filtration, to provide a concentrated antigen, (ii) lyophilising the concentrated antigen, to provide the lyophilised vaccine antigen, and (iii) reconstituting the lyophilised vaccine antigen in an aqueous buffer to provide a reconstituted antigen; wherein the liquid composition is a bulk vaccine.

Apart from the material which is used for reconstitution in step (iii), details for this further aspect are the same as already described herein.

Also disclosed herein is a process in which step (i) is not restricted to using centrifugal filtration. Various techniques can be used for concentration step (i), including but not limited to: centrifugal filtration; ultrafiltration; or tangential flow filtration (also known as crossflow filtration). These three concentration techniques are not mutually exclusive *e.g.* the invention can use tangential flow ultrafiltration.

Tangential flow filtration (TFF) involves passing a liquid tangentially across a filter membrane. The sample side is typically held at a positive pressure relative to the filtrate side. As the liquid flows over the filter, components therein can pass through the membrane into the filtrate. Continued flow causes the volume of the filtrate to increase, and thus the concentration of the antigen in the retentate increases. TFF contrasts with deadend filtration, in which sample is passed through a membrane rather than tangentially to it. Many TFF systems are commercially available. The MWCO of a TFF membrane determines which solutes can pass through the membrane (*i.e*. into the filtrate) and which are retained (*i.e.* in the retentate). The MWCO of a TFF filter used with the invention will be selected such that substantially all of the antigen of interest remains in the retentate.

The concentration step preferably increases the concentration of the antigen of interest by at least *n*-fold, where n is 5, 6, 7, 8, 9, 10, 12, 15, 20, 25, 30, 35, 40, 45, 50, 60, 70, 80 or more.

In this aspect, reconstitution in step (iii) uses a buffer (*e.g.* a phosphate buffer, a Tris buffer, a borate buffer, a succinate buffer, a histidine buffer, or a citrate buffer). Buffers will typically be included in the 5-20mM range. A phosphate buffer is preferred. Reconstitution using water, or using a non-aqueous solvent, is not part of this aspect of the invention.

Step (i) concentrated the a first liquid volume of vaccine antigen, providing a composition with the same amount of antigen in a second (reduced) liquid volume. Step (ii) dried this concentrated material. This dried material is reconstituted in a third volume of buffer. The second volume is lower than the first volume. The third volume is either equal to or, preferably, less than the second volume (and thus, by definition, lower than the first volume *i.e.* the overall process has provided a more concentrated aqueous form of the antigen in the initial liquid composition).

The reconstituted antigen of this aspect can be used to formulate vaccine as already described herein.

This aspect is particularly useful for preparing influenza vaccines, including: monovalent or multivalent vaccines; egg-derived or cell-culture-derived vaccines; inactivated or live vaccines; surface antigen or split virus vaccines; liquid or solid vaccines; *etc.*

In one embodiment the buffer-reconstituted antigen is used to coat microneedles as described above.

### General

The term "comprising" encompasses "including" as well as "consisting" *e.g*. a composition "comprising" X may consist exclusively of X or may include something additional *e.g.* X + Y.

The word "substantially" does not exclude "completely" *e.g.* a composition which is "substantially free" from Y may be completely free from Y. Where necessary, the word "substantially" may be omitted from the definition of the invention.

The term "about" in relation to a numerical value x is optional and means, for example, *x*±5%.

Unless specifically stated, a process comprising a step of mixing two or more components does not require any specific order of mixing. Thus components can be mixed in any order. Where there are three components then two components can be combined with each other, and then the combination may be combined with the third component, *etc.*

Where animal (and particularly bovine) materials are used in the culture of cells, they should be obtained from sources that are free from transmissible spongiform encephalopathies (TSEs), and in particular free from bovine spongiform encephalopathy (BSE). Overall, it is preferred to culture cells in the total absence of animal-derived materials.

Where a compound is administered to the body as part of a composition then that compound may alternatively be replaced by a suitable prodrug.

### BRIEF DESCRIPTION OF THE DRAWINGS

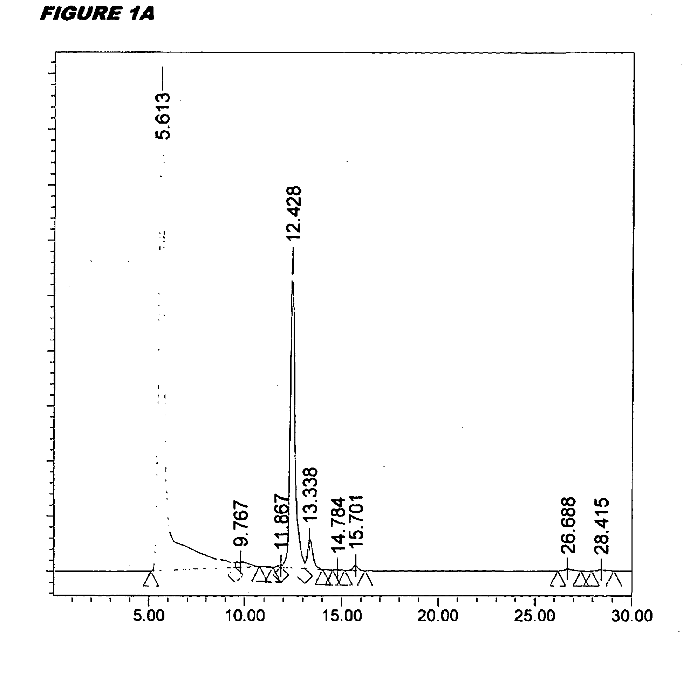

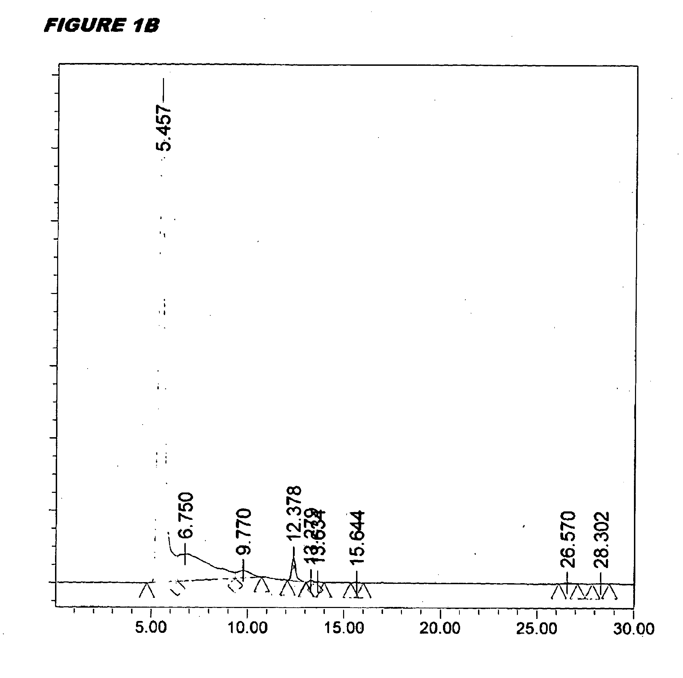

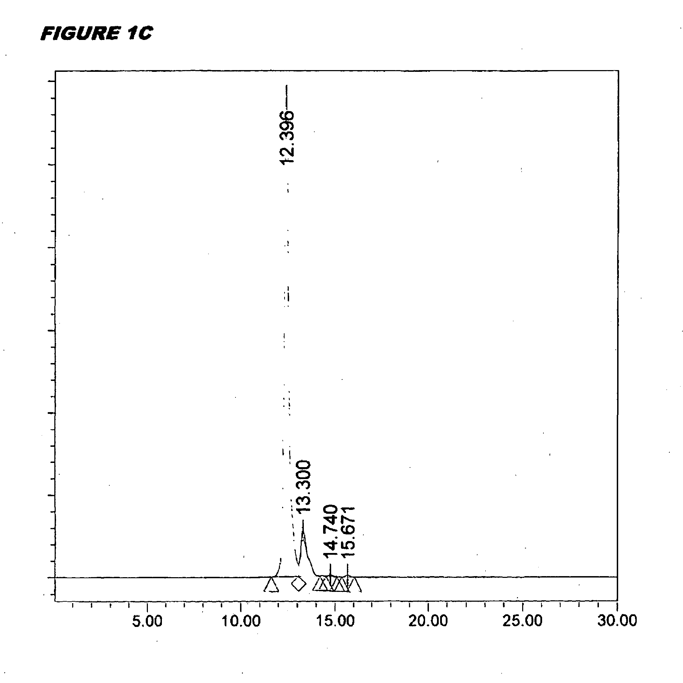

Figure 1 shows SEC chromatographs of centrifugal filtrations. The x-axis shows retention time (minutes) and the y-axis shows absorbance units. Figure 1A shows starting antigen. Figure 1B shows the retentate after 45 minutes. Figure 1C shows the filtrate after 45 minutes.

### MODES FOR CARRYING OUT THE INVENTION

### Centrifugal filtration

Centrifugal filtration used a Millipore™ device with a 10kDa cut-off, operated at 5000rpm.

Three centrifugation durations were tested: 15, 30 and 45 minutes. The retentate (concentrate) and filtrate were checked to see the location of an influenza virus hemagglutinin. Figure 1 shows that the antigen is still in the retentate after 45 minutes. Antigen concentration was 3-fold after 15 minutes, 6-fold after 30 minutes, and 13-fold after 45 minutes. Antigen recovery was 40% after 15 minutes, 41% after 30 minutes, and 55% after 45 minutes. Thus 45 minutes was chosen for further work.

In further work, antigen was lyophilised after centrifugation, to provide further concentration. Sucrose was used as the lyoprotectant, alone (at two different concentrations) or with mannitol. Lyophilised material was reconstituted. The reconstituted samples contained visible aggregates. Relative to the starting material, HA content (measured by ELISA) was concentrated as follows:

| **Treatment** | **Concentration (x)** |
|---|---|
| Starting material | 1.0 x |
| Addition of sucrose | 2.3 x |
| Addition of sucrose (higher concentration) | 1.3 x |
| Addition of sucrose + mannitol | 0.8 x |
| Sucrose, lyophilise, reconstitute | 13.3 x |
| Sucrose+mannitol, lyophilise, reconstitute | 8.5 x |
| Centrifuge, sucrose, lyophilise, reconstitute | 25.2 x |
| Centrifuge, sucrose (higher), lyophilise, reconstitute | 28.4 x |

Thus the combination of centrifugation and lyophilisation can provide a >25-fold concentration in influenza virus HA content. The two centrifuged samples were also assessed by SRID and they showed a 21.1x and 35.1x increase in HA content, with the higher sucrose level again giving better results.

### Ultrafiltration (example provided for reference only)

Ultrafiltration used an Amicon™ stir cell concentrator with a 10kDa cut-off membrane made from regenerated cellulose, operated under pressurised nitrogen for 1 hour.

If a lyophilisation was added, followed by reconstitution back into the pre-lyophilisation volume, the reconstituted material had a HA concentration (as measured by SRID) comparable to the starting material, indicating no loss of functional antigen. The reconstituted material was stable for >2 weeks.

### REFERENCES

[1] Malke et al. (2010) The Internet Journal of Pediatrics and Neonaology. Vol 11, number 2.
[2] Koutsonanos et al. (2009) PLoS ONE 4(e): e4773.
[3] Quan et al. (2009) PLoS ONE 4(9):e7152.
[4] WO2007/030477.
[5] US-6945952.
[6] US-7211062.
[7] US-7182747.
[8] Oh et al. (2006) American Association of Pharmaceutical Scientists, 2006 Annual Meeting and Exposition. The AAPS Journal. 8(S2). (Annual Meeting).
[9] Gill & Prausnitz (2007) J Control Release 117:227-37.
[10] WO2007/124393.
[11] WO2007/061964.
[12] WO2007/059289.
[13] Jin et al. (2009) Biomed Microdevices. 11(6):1195-203.
[14] Su et al. (2009) ACS Nano. 3(11):3719-29.
[15] Cui & Mumper (2002) Pharmaceutical Research 19:947-53.
[16] WO02/051382.
[17] WO2010/002418.
[18] Vaccines. (eds. Plotkin & Orenstein). 4th edition, 2004, ISBN: 0-7216-9688-0.
[19] WO02/28422.
[20] WO02/067983.
[21] WO02/074336.
[22] WO01/21151.
[23] WO02/097072.
[24] WO2005/113756.
[25] Huckriede et al. (2003) Methods Enrymol 373:74-91.
[26] WO96/37624.
[27] WO98/46262.
[28] WO95/18861.
[29] Bright et al. (2008) PLoS ONE 3:e1501.
[30] Crevar & Ross (2008) Virology Journal 5:131.
[31]WO97/37000.
[32] Brands et al. (1999) Dev Biol Strand 98:93-100.
[33] Halperin et al. (2002) Vaccine 20:1240-7.
[34] Tree et al. (2001) Vaccine 19:3444-50.
[35] Kistner et al (1998) Vaccine 16:960-8.
[36] Kistner et al. (1999) Dev Biol Stand 98:101-1-10.
[37] Bruhl et al. (2000) Vaccine 19:1149-58.
[38] Pau et al. (2001) Vaccine 19:2716-21.
[39] WO03/076601.
[40] WO2005/042728.
[41] WO03/043415.
[42] WO01/85938.
[43] WO2006/108846.
[44] EP-A-1260581 (WO01/64846).
[45] WO2006/071563.
[46] WO2005/113758
[47] WO2006/027698.
[48] WO03/023021
[49] WO03/023025
[50] WO97/37001.
[51] EP-B-0870508.
[52] US 5948410.
[53] WO2007/052163.
[54] WO2008/068631.
[55] Rota et al. (1992) J Gen Virol 73:2737-42.
[56] GenBank sequence GI:325176.
[57] Holmes et al. (2005) PLoS Biol. 3(9):e300.
[58] McCullers et al. (1999) J Virol 73:7343-8.
[59] GenBank sequence 01:325237.
[60] Herlocher et al. (2004) J Infect Dis 190(9):1627-30.
[61] Le et al. (2005) Nature 437(7062):1108.
[62] Banzhoff (2000) Immunology Letters 71:91-96.
[63] Lasley (2007) Pediatric Asthma, Allergy & Immunology. 20(3): 201-5.
[64] Coop et al. (2008) Int Arch Allergy Immunol. 146(1):85-8.
[65] WO2009/001217
[66] Treanor et al. (1996) J Infect Dis 173:1467-70.
[67] Keitel et al. (1996) Clin Diagn Lab Immunol 3:507-10.
[68] US 4,680,338.
[69] US 4,988,815.
[70] WO92/15582.
[71] Johnson et al. (1999) Bioorg Med Chem Lett 9:2273-2278.
[72] Evans et al. (2003) Expect Rev Vaccines 2:219-229.
[73] WO03/011223.
[74] Wong et al. (2003) J Clin Pharmacol 43(7):735-42.
[75] US2005/0215517.
[76] Potter & Oxford (1979) Br Med Bull 35: 69-75.

## Claims

1. A process for preparing a lyophilised influenza vaccine antigen, comprising steps of (i) increasing the concentration of hemagglutinin antigen in a liquid composition including that antigen using centrifugal filtration, to provide a concentrated antigen, and (ii) lyophilising the concentrated antigen, to provide the lyophilised vaccine antigen;
wherein the liquid composition is a bulk vaccine.

2. A process for preparing a reconstituted liquid vaccine antigen, comprising steps of: (a) lyophilising an antigen by the process of claim 1; and (b) reconstituting the lyophilised vaccine antigen in an aqueous liquid.

3. The process of claim 2, wherein the volume of the aqueous liquid used in step (b) is lower than the volume of the liquid composition used at the start of step (a).

4. The process of claim 2, wherein the volume of the aqueous liquid used in step (b) is lower than the volume of the concentrated antigen made during step (a).

5. The process of claim 2, claim 3 or claim 4, wherein the reconstituted liquid vaccine antigen is used to formulate a vaccine.

6. The process of any preceding claim, wherein the influenza vaccine is an inactivated influenza vaccine

7. The process of any preceding claim, wherein step (i) increases antigen concentration by at least 10 fold.

8. The process of any preceding claim, wherein the lyophilised vaccine antigen or the reconstituted liquid vaccine antigen is used to prepare a solid vaccine.

9. The process of claim 8, wherein the solid vaccine comprises biodegradable microneedles

10. The process of claim 8, wherein the solid vaccine comprises a coated microneedle.

11. The process of claim 10, comprising applying the reconstituted liquid vaccine antigen to the surface of one or more solid microneedles to provide a coated microneedle device for injection of the vaccine.

12. The process of claim 8, wherein the solid vaccine comprises a thin film.

13. The process of claim 12, comprising mixing the reconstituted liquid antigen with one or more orally-soluble polymers, then forming a film using the mixture to provide a thin film suitable for buccal administration of the vaccine.

14. The process of claim 12, comprising mixing the reconstituted liquid antigen with one or more topically-soluble polymers, then forming a film using the mixture to provide a thin film suitable for transcutaneous administration of the vaccine.

15. A process for preparing a packaged vaccine, comprising: (i) preparing a solid vaccine by the process of any one of claims 8 to 14; then (ii) packaging a solid vaccine into an individual unit dose pouch.

16. A process for preparing an influenza vaccine antigen, comprising steps of (i) increasing the concentration of hemagglutinin antigen in a liquid composition including that antigen using centrifugal filtration, to provide a concentrated antigen, (ii) lyophilising the concentrated antigen, to provide the lyophilised vaccine antigen, and (iii) reconstituting the lyophilised vaccine antigen in an aqueous buffer to provide a reconstituted antigen;
wherein the liquid composition is a bulk vaccine.

## Patentansprüche

1. Verfahren zur Herstellung eines lyophilisierten Influenza-Antigens zur Impfung, umfassend die Schritte von (i) Erhöhung der Konzentration von Hämagglutinin-Antigen in einer flüssigen Zusammensetzung, die dieses Antigen enthält, unter Verwendung von Zentrifugalfiltration, zur Bereitstellung eines konzentrierten Antigens, und (ii) Lyophilisierung des konzentrierten Antigens, zur Bereitstellung des lyophilisierten Antigens zur Impfung;
worin die flüssige Zusammensetzung ein Bulk-Impfstoff ist.

2. Verfahren zur Herstellung eines rekonstituierten flüssigen Antigens zur Impfung, umfassend die Schritte von: (a) Lyophilisierung eines Antigens durch das Verfahren nach Anspruch 1; und (b) Rekonstitution des lyophilisierten Antigens zur Impfung in einer wässrigen Flüssigkeit.

3. Verfahren nach Anspruch 2, worin das Volumen der in Schritt (b) verwendeten wässrigen Flüssigkeit geringer als das Volumen der flüssigen Zusammensetzung ist, die zu Beginn von Schritt (a) verwendet wurde.

4. Verfahren nach Anspruch 2, worin das Volumen der in Schritt (b) verwendeten wässrigen Flüssigkeit geringer als das Volumen des konzentrierten Antigens ist, das in Schritt (a) hergestellt wurde.

5. Verfahren nach Anspruch 2, Anspruch 3 oder Anspruch 4, worin das rekonstituierte flüssige Antigen zur Impfung zur Formulierung eines Impfstoffs verwendet wird.

6. Verfahren nach einem der vorhergehenden Ansprüche, worin der Influenza-Impfstoff ein inaktivierter Influenza-Impfstoff ist.

7. Verfahren nach einem der vorhergehenden Ansprüche, worin Schritt (i) die Antigen-Konzentration mindestens 10-fach erhöht.

8. Verfahren nach einem der vorhergehenden Ansprüche, worin das lyophilisierte Antigen zur Impfung oder das rekonstituierte flüssigen Antigen zur Impfung zur Herstellung eines festen Impfstoffs verwendet wird.

9. Verfahren nach Anspruch 8, worin der feste Impfstoff biologisch abbaubare Mikronadeln umfasst.

10. Verfahren nach Anspruch 8, worin der feste Impfstoff eine beschichtete Mikronadel umfasst.

11. Verfahren nach Anspruch 10, umfassend die Applikation des rekonstituierten flüssigen Antigens zur Impfung auf die Oberfläche von einer oder mehreren festen Mikronadeln zur Bereitstellung einer beschichteten Mikronadelvorrichtung zur Injektion des Impfstoffs.

12. Verfahren nach Anspruch 8, worin der feste Impfstoff eine dünne Folie umfasst.

13. Verfahren nach Anspruch 12, umfassend Vermischen des rekonstituierten flüssigen Antigens mit einem oder mehreren oral löslichen Polymeren, dann Formen einer Folie unter Verwendung des Gemisches zur Bereitstellung einer dünnen Folie, die zur bukkalen Verabreichung des Impfstoffs geeignet ist.

14. Verfahren nach Anspruch 12, umfassend Vermischen des rekonstituierten flüssigen Antigens mit einem oder mehreren topisch löslichen Polymeren, dann Formen einer Folie unter Verwendung des Gemisches zur Bereitstellung einer dünnen Folie, die zur transkutanen Verabreichung des Impfstoffs geeignet ist.

15. Verfahren zur Herstellung eines verpackten Impfstoffs, umfassend: (i) Herstellung eines festen Impfstoffs durch das Verfahren nach einem der Ansprüche 8 bis 14; dann (ii) Verpackung eines festen Impfstoffs in einen individuellen Einheitsdosis-Beutel.

16. Verfahren zur Herstellung eines Influenza-Antigens zur Impfung, umfassend die Schritte von (i) Erhöhung der Konzentration von Hämagglutinin-Antigen in einer flüssigen Zusammensetzung, die dieses Antigen enthält, unter Verwendung von Zentrifugalfiltration, zur Bereitstellung eines konzentrierten Antigens, (ii) Lyophilisierung des konzentrierten Antigens, zur Bereitstellung des lyophilisierten Antigens zur Impfung, und (iii) Rekonstitution des lyophilisierten Antigens zur Impfung in einem wässrigen Puffer zur Bereitstellung eines rekonstituierten Antigens;
worin die flüssige Zusammensetzung ein Bulk-Impfstoff ist.

## Revendications

1. Processus de préparation d'un antigène de vaccin antigrippal lyophilisé, comprenant les étapes consistant à (i) augmenter la concentration en antigène hémagglutinique dans une composition liquide comprenant cet antigène, en utilisant une filtration centrifuge, pour fournir un antigène concentré, et (ii) lyophiliser l'antigène concentré, pour fournir l'antigène vaccinal lyophilisé ;
dans lequel la composition liquide est un vaccin brut.

2. Processus de préparation d'un antigène vaccinal liquide reconstitué, comprenant les étapes consistant à : (a) lyophiliser un antigène par le processus de la revendication 1 ; et (b) reconstituer l'antigène vaccinal lyophilisé dans un liquide aqueux.

3. Processus selon la revendication 2, dans laquelle le volume du liquide aqueux utilisé dans l'étape (b) est inférieur au volume de la composition liquide utilisée au début de l'étape (a).

4. Processus selon la revendication 2, dans laquelle le volume du liquide aqueux utilisé dans l'étape (b) est inférieur au volume de l'antigène concentré, élaboré pendant l'étape (a).

5. Processus selon la revendication 2, la revendication 3 ou la revendication 4, dans laquelle l'antigène vaccinal liquide reconstitué est utilisé pour formuler un vaccin.

6. Processus selon l'une quelconque des revendications précédentes, dans laquelle le vaccin antigrippal est un vaccin antigrippal inactivé.

7. Processus selon l'une quelconque des revendications précédentes, dans laquelle l'étape (i) augmente la concentration en antigène d'au moins 10 fois.

8. Processus selon l'une quelconque des revendications précédentes, dans laquelle l'antigène vaccinal lyophilisé ou l'antigène vaccinal liquide reconstitué est utilisé pour préparer un vaccin solide.

9. Processus selon la revendication 8, dans laquelle le vaccin solide comprend des micro-aiguilles biodégradables.

10. Processus selon la revendication 8, dans laquelle le vaccin solide comprend une micro-aiguille enduite.

11. Processus, selon la revendication 10, comprenant une application de l'antigène vaccinal liquide reconstitué à la surface d'une ou plusieurs micro-aiguille(s) solide(s) pour fournir un dispositif à micro-aiguille(s) enduite(s) pour une injection du vaccin.

12. Processus selon la revendication 8, dans laquelle le vaccin solide comprend une fine pellicule.

13. Processus, selon la revendication 12, comprenant les étapes consistant à mélanger l'antigène liquide reconstitué avec un ou plusieurs polymère(s) soluble(s) oralement, puis former une pellicule en utilisant le mélange pour fournir une fine pellicule appropriée pour une administration buccale du vaccin.

14. Processus, selon la revendication 12, comprenant les étapes consistant à mélanger l'antigène liquide reconstitué avec un ou plusieurs polymère(s) soluble(s) par voie topique, puis former une pellicule en utilisant le mélange pour fournir une fine pellicule appropriée pour une administration transcutanée du vaccin.

15. Processus de préparation d'un vaccin conditionné, comprenant les étapes consistant à : (i) préparer un vaccin solide par le processus selon l'une quelconque des revendications 8 à 14 ; puis (ii) conditionner un vaccin solide dans une pochette à dose unitaire individuelle.

16. Processus de préparation d'un antigène de vaccin antigrippal, comprenant les étapes consistant à (i) augmenter la concentration en antigène hémagglutinique dans une composition liquide comprenant cet antigène, en utilisant une filtration centrifuge, pour fournir un antigène concentré, (ii) lyophiliser l'antigène concentré, pour fournir l'antigène vaccinal lyophilisé, et (iii) reconstituer l'antigène vaccinal lyophilisé dans un tampon aqueux pour fournir un antigène reconstitué ;
dans lequel la composition liquide est un vaccin brut.
